Europäisches Patentamt

European Patent Office

Office européen des brevets

(19)

(11) Numéro de publication: **0 366 528**
**A1**

(12) **DEMANDE DE BREVET EUROPEEN**

(21) Numéro de dépôt: 89402898.4

(22) Date de dépôt: **20.10.89**

(51) Int. Cl.5: **A61B 6/00 , A61B 6/03**

(30) Priorité: **28.10.88 FR 8814166**

(43) Date de publication de la demande:
**02.05.90 Bulletin 90/18**

(84) Etats contractants désignés:
**DE ES GB IT NL**

(71) Demandeur: **GENERAL ELECTRIC CGR S.A.**
**100, rue Camille-Desmoulins**
**F-92130 Issy les Moulineaux(FR)**

(72) Inventeur: **Feldman, Andréi**
**Cabinet Ballot-Schmit 7, rue Le Sueur**
**F-75116 Paris(FR)**
Inventeur: **Cornuejols, Dominique**
**Cabinet Ballot-Schmit 7, rue Le Sueur**
**F-75116 Paris(FR)**

(74) Mandataire: **Ballot, Paul Denis Jacques**
**Cabinet Ballot-Schmit 7, rue le Sueur**
**F-75116 Paris(FR)**

(54) **Procédé et système d'étalonnage d'un scanner à rayons X à partir de l'image d'au moins un étalon.**

(57) Procédé et système d'étalonnage d'un scanner à rayons X, caractérisé par le fait que l'on calcule d'abord les longueurs $d_{ij}$ (57) des trajets des rayons X à l'aide de l'image de l'étalon; on calcule ensuite les atténuations théoriques $Ac_{ij}$ (58) ainsi que les atténuations $Am_{ij}$ (59) mesurées sur l'image. On en déduit alors le coefficient de proportionnalité K entre les valeurs $Ac_{ij}$ et $Am_{ij}$ pour obtenir des valeurs de $K.Ac_i$. Les valeurs $KAc_i$ et $Am_i$ sont utilisés pour calculer (66 et 68) les corrections à effectuer par canal en fonction de l'atténuation.

FIG_6

EP 0 366 528 A1

## PROCEDE ET SYSTEME D'ETALONNAGE D'UN SCANNER A RAYONS X A PARTIR DE L'IMAGE D'AU MOINS UN ETALON

L'invention concerne les scanners à rayons X et plus particulièrement un procédé d'étalonnage d'appareils de ce type qui utilise l'image d'un ou plusieurs étalons ; elle concerne également un système de mise en oeuvre dudit procédé.

Pour examiner un patient, on utilise de plus en plus des appareils à rayons X appelés "scanners" qui réalisent des images de coupes transversales du patient. Ces appareils sont basés sur le phénomène physique d'absorption des rayons X par le corps humain. Cette absorption est directement liée à la distance parcourue x des rayons X dans le corps selon la formule :

$$I = I_o e^{-bx}$$

formule dans laquelle :

$I_o$ est l'intensité du rayonnement entrant dans le corps humain, I est l'intensité du rayonnement sortant du corps humain

b est un coefficient d'atténuation qui dépend du corps traversé.

Dans une échelle de mesure logarithmique, l'atténuation $I/I_o$ est égale à bx, c'est-à-dire qu'elle est proportionnelle à la distance x.

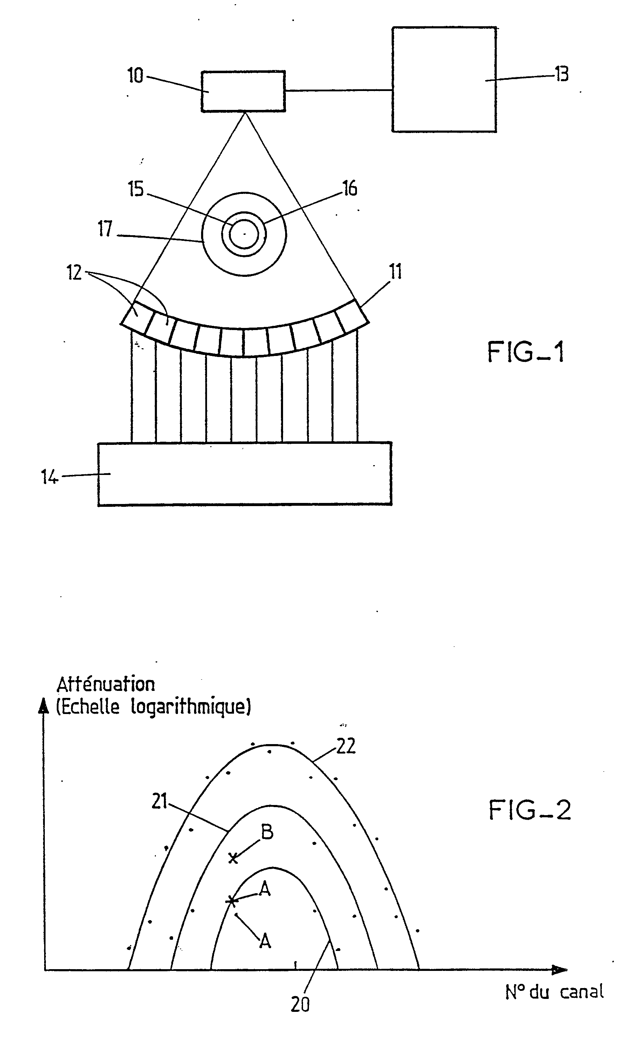

Ces appareils sont constitués essentiellement, comme le montre la figure 1, d'une source 10 de rayons X associée à un dispositif de détection 11, ces deux éléments étant disposés l'un par rapport à l'autre dans une relation géométrique fixe de manière à pouvoir intercaler entre eux le corps à examiner. En outre, ils sont supportés par une structure (non représentée) qui peut tourner autour du corps à examiner de manière à irradier le corps suivant des angles différents. La source à rayons X, qui est commandée par un dispositif 13, émet ses rayons suivant un secteur angulaire qui a une largeur suffisante pour illuminer toute la section transversale du corps. Le dispositif de détection 11 a la forme d'un secteur annulaire dont la longueur est adaptée à la largeur du faisceau de rayons X et est constitué d'un grand nombre de détecteurs élémentaires 12 juxtaposés les uns à côté des autres.

Pour obtenir une image de la section transversale du corps humain traversé par le faisceau de rayons X, on fait tourner la structure de support de la source 10 et du dispositif de détection 11 autour du corps et on mesure les signaux de sortie des détecteurs élémentaires 12 pour les traiter de manière appropriée selon des procédés connus afin d'en tirer une image représentative de la section transversale. Pour ce traitement, les détecteurs élémentaires 12, également appelés canaux, sont connectés à un dispositif électronique 14 qui effectue en premier lieu un calcul du logarithme des signaux reçus de manière à obtenir un signal dont l'amplitude est proportionnelle à l'atténuation des rayons X.

Par suite de l'effet de différents phénomènes qui ne seront pas expliqués ici, l'amplitude de ce signal pour chaque détecteur élémentaire ou canal n'est pas proportionnelle à l'atténuation effectivement subie. En conséquence, pour remédier à cet inconvénient, il a été imaginé divers procédés qui consistent par exemple à relever les signaux de sortie des canaux en présence de corps dont on connaît les dimensions et le coefficient d'absorption, de manière à calculer les atténuations (calculs de logarithmes) et à comparer ces atténuations mesurées à des valeurs calculées en fonction des dimensions et du coefficient d'absorption du corps ou étalon. Ces comparaisons permettent de déduire une loi de correspondance ou une loi modificatrice entre les valeurs mesurées et les valeurs que l'on devrait obtenir. Cette loi peut être sous la forme de fichiers de correspondance ou de formules mathématiques traduisant cette correspondance pour chaque canal de détection.

Les étalons qui sont utilisés pour effectuer ces mesures dites d'étalonnage sont par exemple des cales d'épaisseurs différentes qui sont introduites à proximité de la source de rayons X, ce qui implique des manipulations au niveau de la source pour introduire et retirer ces cales. En outre, la forme de ces cales et leur position sont éloignées de la forme et de la position du corps du malade à examiner, ce qui augmente la non-linéarité du système.

Dans le brevet américain N° 4352020, il est proposé d'utiliser des cales circulaires 15 à 18, de différents diamètres, qui sont disposées au centre de rotation de la structure du support. Ceci permet de se rapprocher des conditions des mesures qui seront effectuées sur le corps à examiner. Ce brevet propose également d'utiliser un étalon en forme de cône à section circulaire qui est déplacé transversalement par rapport au faisceau de manière à obtenir différentes longueurs d'atténuation. Avec les étalons décrits, les mesures sont effectuées pour une position déterminée de la structure de support et par étalon.

La figure 2 montre l'allure de trois courbes de réponse 20, 21 et 22 de l'atténuation en fonction de la position des canaux dans le cas de mesures sur trois étalons de forme circulaire. Les valeurs mesurées sont représentées par les pointillés et varient autour d'une valeur moyenne qui représente la valeur théorique dans un système linéaire. Ces courbes peuvent être utilisées de la manière suivante ; lorsque le signal mesuré correspond à un point A, on en déduira que le signal linéaire est le point A' de la courbe moyenne 20. Lorsque le signal mesuré correspond à un point B situé entre les courbes 20 et 21, on en déduira le signal linéaire par interpolation entre les courbes 20 et 21. Cette interpolation peut être calculée suivant une loi linéaire ou plus généralement une loi polynomiale.

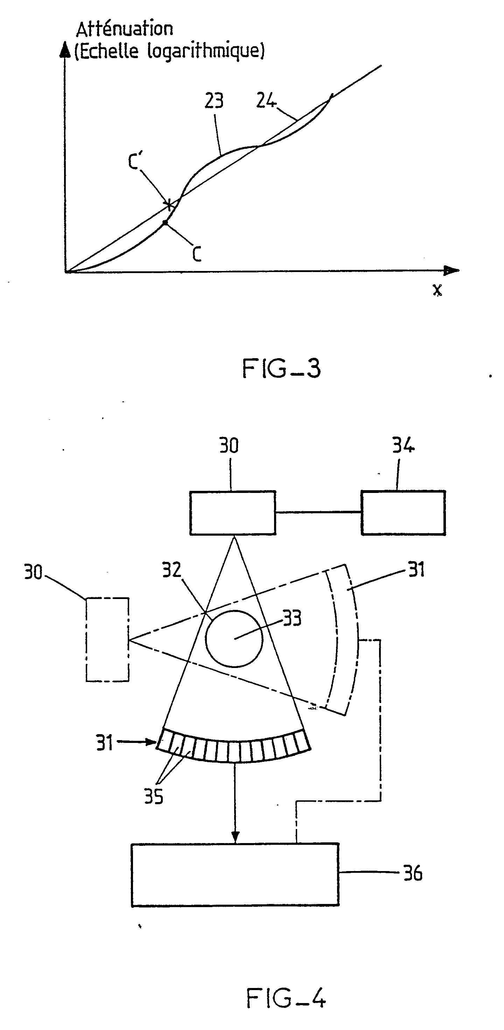

Les courbes 23 et 24 de la figure 3 montrent sous une autre forme le principe de l'étalonnage au niveau d'un canal. Il s'agit de courbes décrivant, dans un canal donné, l'atténuation en fonction de l'épaisseur x pour des valeurs mesurées (courbe 23) et pour des valeurs calculées (droite 24) : en fait, les valeurs mesurées donnent des points, points que l'on relie entre eux selon une loi choisie linéaire ou polynomiale, de manière à obtenir une courbe continue. Lorsqu'on mesure une atténuation, cela correspond par exemple au point C de la courbe 23 et on en déduit la valeur linéaire correspondant au point C' de la courbe 24.

Le brevet américain précité décrit un appareil dans lequel la correspondance entre les valeurs mesurées et les valeurs réelles d'atténuation est effectuée par un système de fichiers crées pendant l'opération d'étalonnage. En ce qui concerne l'interpolation, le brevet propose des interpolations linéaires, cubiques et biquadratiques mais seule l'interpolation linéaire est décrite de manière détaillée.

Les procédés d'étalonnage qui ont été succinctement décrits ci-dessus, sont caractérisés par le fait qu'ils effectuent un traitement sur les signaux mesurés afin d'obtenir une image exempte d'artéfacts, c'est-à-dire sans défauts. Cependant, l'obtention d'une telle image n'est pas toujours aisée, notamment parce que les calculs sont effectués avec certaines approximations qui, dans certains cas, peuvent être grossières.

En conséquence, un but de la présente invention est de mettre en oeuvre un procédé qui traite, non pas les signaux mesurés eux-mêmes, mais l'image obtenue de manière à éliminer les artéfacts circulaires.

Un autre but de la présente invention est un système qui permet de mettre en oeuvre ledit procédé.

L'invention se rapporte à un procédé d'étalonnage d'un scanner à rayons X qui comporte une source de rayonnement X et un dispositif de détection à N canaux, caractérisé en ce qu'il comprend les opérations suivantes :

- La mise en place d'un premier étalon circulaire entre la source de rayonnement X (30) et le dispositif de détection (31),
- la réalisation d'un tour de scanner correspondant à m vues distinctes
- la reconstruction de l'image (81) de l'étalon (32) de manière à obtenir une image matricielle indiquant pour chaque point de coordonnées x et y, la valeur de la brillance du point élémentaire d'image ou pixel,
- le calcul, à l'aide de l'image matricielle, des N.m longueurs $d_{ij}$ des trajets des rayons X pour les N canaux et les m vues;
- le calcul des atténuations théoriques et fictives $AC_{ij}$, au niveau de l'image pour les trajets $d_{ij}$ en supposant que la brillance a une valeur fixe et uniforme pour chaque pixel;
- la mesure, à l'aide de l'image matricielle, des atténuations $Am_{ij}$ pour les mêmes trajets $d_{ij}$ en tenant compte des valeurs de brillance des pixels;
- le calcul, par canal, des valeurs moyennes des atténuations $Ac_i$ et $Am_i$
- le calcul d'un facteur de proportionnalité K entre les valeurs $Ac_i$ et $Am_i$, de sorte que

$$K \sum_{i=1}^{N} Ac_i = \sum_{i=1}^{N} Am_i$$

- le calcul des atténuations $KAc_i$ et
- le calcul, à partir des valeurs $KAc_i$ et $Am_i$, des corrections à effectuer par canal en fonction de l'atténuation.

L'invention se rapporte également à un système pour mettre en oeuvre le procédé caractérisé en ce qu'il comprend :
- des moyens pour réaliser une image matricielle de l'étalon sous forme de coordonnées x et y de pixels et de valeurs de brillance desdits pixels,
- des premiers moyens de calcul pour calculer, à partir de l'image matricielle de l'étalon, N.m longueurs $d_{ij}$ des trajets des rayons X pour les N canaux et les m vues, ainsi que les valeurs $Ac_{ij}$,
- des deuxièmes moyens de calcul pour calculer les valeurs d'atténuation $Am_{ij}$ pour les longueurs $d_{ij}$,
- des troisièmes moyens de calcul pour calculer, d'une part, le coefficient de proportionnalité K entre les valeurs $Ac_i$ et $Am_i$, et, d'autre part, les valeurs $K.Ac_i$, et
- des moyens d'enregistrement pour enregistrer les valeurs $K.Ac_i$ et $Am_i$, et
- des moyens pour calculer, à partir des valeurs $K.Ac_i$ et $Am_i$, les corrections à effectuer par canal en fonction de l'atténuation.

D'autres caractéristiques et avantages de la présente invention apparaîtront à la lecture de la description suivante d'un exemple particulier de réalisation, ladite description étant faite avec les dessins joints dans lesquels :

- la figure 1 est un schéma de principe d'un scanner à rayons X dans lequel l'étalonnage est obtenu à l'aide d'étalons circulaires,

- la figure 2 est un diagramme montrant différentes courbes d'atténuation en fonction de la position des détecteurs ou canaux et du diamètre de l'étalon,

- la figure 3 est un diagramme montant les courbes d'atténuation théorique et mesurée pour un détecteur ou canal déterminé,

- la figure 4 est un schéma de principe d'un scanner à rayons X qui montre deux positions du scanner autour d'un étalon circulaire,

- la figure 5 est un diagramme géométrique montrant l'image d'un étalon circulaire sur un dispositif de visualisation,

- la figure 6 est un schéma fonctionnel d'un système de traitement des signaux lus pour mettre en oeuvre le procédé d'étalonnage selon l'invention, et

- la figure 7 est un dessin géométrique montrant les positions relatives de l'étalon par rapport au scanner.

Les figures 1, 2 et 3, qui ont permis de décrire l'art antérieur dans le préambule, ne seront pas décrites à nouveau.

Comme on l'a indiqué précédemment, le procédé selon l'invention réalise l'étalonnage en utilisant l'image de l'étalon obtenue par le scanner. Cette image, qui correspond à des valeurs mesurées de l'atténuation contenant des défauts, est utilisée pour calculer des valeurs théoriques d'atténuation qui sont comparées aux valeurs mesurées pour obtenir des valeurs de correction de ces dernières. En d'autres mots, c'est l'image de l'étalon que l'on corrige par calcul pour en faire disparaître les défauts. Les corrections ainsi calculées seront celles qui seront utilisées pour modifier les valeurs mesurées sur un patient. Pour obtenir des corrections selon la longueur du trajet des rayons X dans le corps du patient, il est nécessaire de calculer les corrections pour des étalons ayant des diamètres différents.

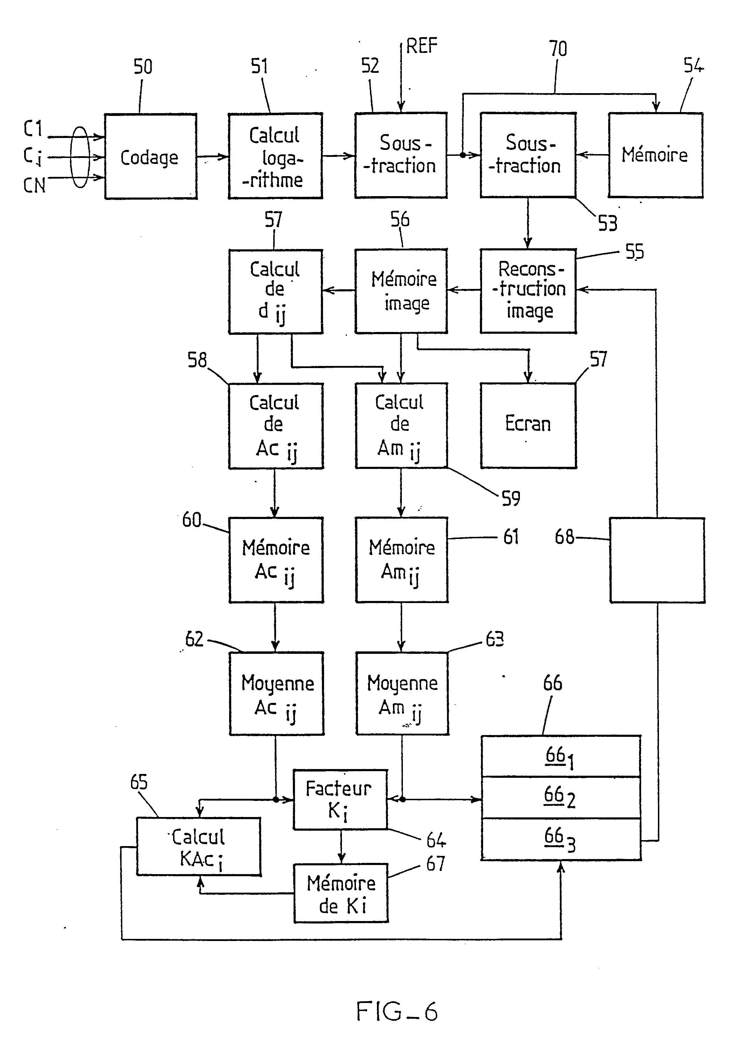

Le schéma de la figure 4 est analogue à celui de la figure 1, en ce sens qu'il montre un scanner à rayons X qui comprend une source 30 à rayons X et un dispositif de détection 31 comportant N détecteurs ou canaux 35 entre lesquels est interposé, en fonctionnement normal, le corps d'un patient ou un étalon 32 de forme circulaire lors des opérations d'étalonnage. Bien que non représentée, une structure est prévue pour supporter la source 30 et le dispositif 31 et faire tourner l'ensemble autour du corps du patient ou de l'étalon, l'axe de rotation étant matérialisé par le point 33. Dans la plupart des procédés d'étalonnage de l'art antérieur, le centre de l'étalon circulaire doit coïncider avec l'axe de rotation 33.Cette condition n'est pas nécessaire dans le procédé selon l'invention. La source à rayons X est commandée par un dispositif de commande 34 tandis que les différents détecteurs ou canaux 35 du dispositif 31 sont connectés à un système électronique 36 qui sera décrit en relation avec la figure 6. Par ailleurs, des moyens, non représentés, sont prévus pour faire tourner l'ensemble source 30-détecteur 31, de sorte qu'il puisse prendre un certain nombre de positions angulaires déterminées $\alpha_j$ au nombre de m, notamment celles représentées sur la figure 4. Dans la suite de la description, on appellera "vue" l'ensemble des N signaux recueillis sur les N détecteurs du dispositif de détection 31 pour une position angulaire $\alpha_j$, $\alpha_j$ correspondant par exemple à l'inclinaison angulaire par rapport à l'horizontale passant par le point 33.

Le procédé d'étalonnage selon l'invention consiste d'abord à effectuer une série de m vues $V_1$... $V_j$... $V_m$ en l'absence d'étalon, opération qui est appelée "acquisition dans l'air". Ces vues servent de référence pour mesurer les atténuations lorsque l'étalon ou le corps du patient est en place. Cette première opération sera mieux comprise en se référant au schéma fonctionnel de la figure 6. Les signaux fournis par les N détecteurs ou canaux $C_1$...$C_i$...$C_N$ sont appliqués à un circuit de codage analogique/numérique 50 qui fournit des valeurs numériques ou codes. Ce sont sur ces codes que seront effectuées toutes les opérations qui vont être décrites ci-après, ces opérations pouvant être réalisées par un calculateur convenablement programmé.

Les N codes résultant d'une mesure à une position élémentaire $\alpha_j$ ou vue sont appliqués à un circuit de calcul de logarithme 51. Les valeurs logarithmiques sont soustraites dans un circuit 52 à une valeur logarithmique de référence REF fournie par un détecteur, appelé moniteur, qui reçoit directement le rayonnement X sans atténuation quelle que soit la position angulaire $\alpha_j$. Ce sont ces N valeurs différentielles, correspondant chacune à un canal, qui donnent les valeurs d'acquisition dans l'air pour une vue. Ces N valeurs sont enregistrées dans la mémoire 54 lors de l'opération d'acquisition par l'intermédiaire de la liaison directe 70. Cette opération est répétée pour chacune des m vues et la valeur obtenue pour chaque canal est additionnée à la précédente pour obtenir une valeur moyenne qui remplace dans la mémoire 54 la valeur moyenne qui résulte des précédentes vues. Après m vues, la mémoire 54 contient N valeurs moyennes, une par canal, qui servent de référence pendant les autres opérations d'étalonnage et pendant les opérations de mesure sur un patient.

Lorsque cette première opération d'acquisition dans l'air, qui est connue par ailleurs, a été réalisée, un premier étalon circulaire 32 est mis en place et on effectue une série de m vues et les signaux correspondant à chaque vue sont traités de la manière suivante. Après traitement dans les circuits 50, 51 et 52 comme expliqué précédemment, les N codes d'une vue sont appliqués à un deuxième circuit de soustraction 53 où ils sont soustraits aux N codes de la valeur moyenne sans étalon contenus dans la mémoire 54. Par cette soustraction, on s'affranchit des caractéristiques des détecteurs 35 qui sont différentes d'un détecteur à l'autre. On remarquera que la première soustraction dans le circuit 52 permet de s'affranchir, notamment, des variations d'émission de la source 30.

Les N.m codes fournis par le deuxième circuit de soustraction 53 sont fournis à un dispositif de reconstruction de l'image 55 qui, selon un procédé connu, réalise une image représentative de l'étalon circulaire 32 mis en place lors de l'étalonnage ou d'une section transversale du corps du patient lors du fonctionnement normal du scanner. Cette image est par exemple représentée par l'intermédiaire d'un écran 57 constitué par exemple d'une matrice de points élémentaires ou pixels organisée en lignes et colonnes; elle est enregistrée dans une mémoire image 56 sous la forme d'un système de coordonnées x et y des pixels, à chaque coordonnée étant associée une valeur de brillance du pixel suivant une échelle déterminée.

Cette image représentative de l'étalon circulaire 32 présente des artéfacts dus aux non-linéarités du système. C'est à partir des images de plusieurs étalons que sont calculées les corrections selon le procédé le l'invention.

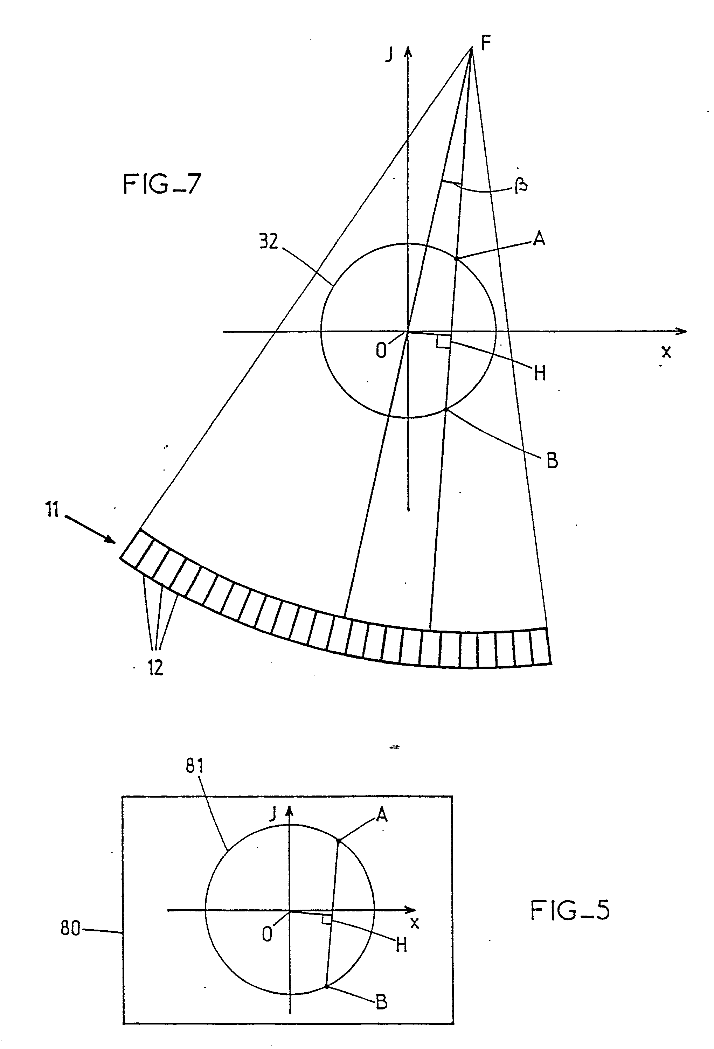

Sur les figures 5 et 7, le segment AB représente le trajet d'un rayon X dans l'étalon 32. Le principe du procédé et du système selon l'invention est alors le suivant. La longueur AB est mesurée à l'aide des coordonnées x et y de A et de B; s'agissant d'un trajet dans un étalon dont l'absorption est fixe et uniforme, on calcule l'atténuation théorique et fictive Ac subie par le rayonnement X sur le trajet géométrique AB. Par ailleurs, on mesure l'atténuation mesurée Am à l'aide des valeurs de brillance des pixels sur le trajet AB telles qu'enregistrées dans la mémoire 56. En l'absence d'artéfacts, ces deux atténuations Ac et Am doivent être égales à un facteur K près; si elles ne le sont pas, une correction doit être effectuée. La détermination de cette correction s'effectue pour chaque canal; à cet effet, les atténuations Ac et Am sont respectivement calculées pour différentes orientations du segment AB correspondant à une même distance OH, chaque orientation correspondant elle-même à une position angulaire déterminée du scanner, c'est-à-dire une vue.

Par construction géométrique du scanner, on remarque que le rayonnement sur les différents trajets AB à la même distance OH = d est toujours détecté par le même canal. Corollairement, pour une autre distance OH, le segment AB aura une longueur différente et le rayonnement sera détecté par un autre canal. En d'autres termes, chaque canal correspondra à une certaine distance OH qui est déterminée par la relation trigonométrique suivante (figure 7).

$$OH = OF \sin \beta$$

sachant que d'un canal au suivant, l'angle $\beta$ varie d'une valeur fixe $\theta$ par construction du scanner. Ces remarques conduisent à ne considérer que les trajets AB qui correspondent à des distances OH associées à un angle $\beta$ relatif à un canal déterminé, ce qui signifie que l'on considère N trajets AB quand il y a N canaux.

Avec un seul étalon, on ne peut déterminer qu'une correction par canal, ce qui est insuffisant. Aussi, l'invention prévoit un procédé dans lequel on utilise plusieurs étalons circulaires, ce qui permet de déterminer autant de corrections par canal en fonction de la longueur du trajet AB.

Ces principes de correction selon l'invention étant exposés, leur mise en oeuvre sera mieux comprise à l'aide de la description des autres éléments non encore décrits du schéma de la figure 6.

Les informations contenues dans la mémoire image 56 sont traitées de la manière suivante dans les dispositifs 57 à 68. D'abord, le dispositif 57 réalise l'opération de calcul des trajets AB = d sur l'image en fonction, d'une part, de la distance OH, cette dernière variant en fonction du pas angulaire $\theta$ entre deux canaux consécutifs, et, d'autre part, de l'orientation correspondant à une position angulaire $\alpha_j$ du scanner, c'est-à-dire à une vue ($\alpha_j$ est l'angle entre les demi-droites OF et Ox Figure 7). On comprend que le résultat de ce calcul peut être sous la forme d'un fichier qui indique pour chaque angle $\alpha_j$ les trajets AB correspondant à chaque canal $C_i$. Ce fichier contient donc N.m valeurs $d_{ij}$.

Le contenu de ce fichier sert à calculer les atténuations théoriques et fictives (Ac) et mesurées (Am) dont il a été question ci-dessus et ceci pour chaque trajet AB.

Le calcul de Ac découle directement du fichier des valeurs $d_{ij}$ en multipliant ces dernières par le coefficient d'absorption de l'étalon 32 théorique exprimé en unités Housfield, dans un circuit 58. On obtient ainsi N.m valeurs théoriques et fictives $Ac_{ij}$ qui constituent des mesures de l'atténuation, valeurs qui sont enregistrées dans une mémoire 60 organisée en lignes et colonnes, chaque ligne correspondant à une vue (angle $\alpha_j$) et chaque colonne correspondant à un canal $C_i$.

Le calcul de Am est effectué dans un circuit 59 à l'aide du fichier des trajets $d_{ij}$ et des informations de brillance des pixels contenues dans la mémoire image 56. Plus précisément, on détermine les pixels qui réalisent chaque trajet $d_{ij}$ et on calcule la somme des brillances de ces pixels, ce qui constitue une mesure de l'atténuation $Am_{ij}$. Les N.m valeurs $Am_{ij}$ sont enregistrées dans une mémoire 61 qui est organisée de la même manière que la mémoire 60.

Comme on l'a indiqué ci-dessus, en l'absence de tout défaut, les valeurs $Ac_{ij}$ et $Am_{ij}$ devraient être égales deux à deux à un facteur de proportionnalité K près. Les écarts entre ces valeurs, après introduction du facteur de proportionnalité, sont donc une mesure des corrections à effectuer sur les mesures pour éliminer les défauts.

Ce facteur de proportionnalité K est déterminé à l'aide du contenu des mémoires 60 et 61. Pour cela, on effectue dans un circuit 62 la moyenne arithmétique des valeurs contenues dans chaque colonne de la mémoire 60 de manière à obtenir N valeurs $Ac_i$ et on fait de même dans un circuit 63 pour obtenir N valeurs moyennes $Am_i$ des valeurs contenues dans chaque colonne de la mémoire 61. Les valeurs moyennes sont ensuite comparées deux à deux dans un circuit 64 pour obtenir N facteurs de proportionnalité $K_i$ tels que :

$$K_i = \frac{Am_i}{Ac_i}$$

Enfin, on obtient le facteur K en effectuant la moyenne des N valeurs $K_i$ dans un circuit 67. C'est ce facteur K, qui résulte d'une moyenne générale, qui est utilisé dans un circuit 65 pour modifier les valeurs moyennes $Ac_i$ et obtenir les valeurs $K.Ac_i$ qui sont comparables aux valeurs moyennes $Am_i$. Les valeurs moyennes $KAc_i$ et $Am_i$ sont enregistrées dans une mémoire 66. On obtient ainsi pour chaque canal une correspondance entre une valeur mesurée $Am_i$ et une valeur théorique $K.Ac_i$ et ceci pour un certaines trajet $d_i$ dans l'étalon circulaire 32 correspondant à un canal $C_i$.

Pour un même canal $C_i$, il est souhaitable d'avoir d'autres trajets $d_i$ de manière à introduire des corrections plus précises. A cet effet, on répète les opérations décrites ci-dessus en relation avec la figure 6 avec d'autres étalons circulaires ayant des diamètres différents. Ceci permet d'enregistrer dans la mémoire 66 d'autres ensembles de couples de valeurs de correspondance pour chaque canal pour d'autres trajets $d_i$.

Ainsi, dans la mémoire 66, la partie $66_1$ est affectée à l'ensemble de couples de valeurs de correspondance obtenu avec le premier étalon 15 (figure 1), la partie $66_2$ est affectée au deuxième étalon 16 et la partie $66_3$ est affectée au troisième etalon 17.

Les informations contenues dans 66 sont utilisées dans un circuit 68 pour étalonner la machine, c'est-à-dire que l'on utilisera ces informations pour calculer une correction de l'atténuation.

Le procédé d'étalonnage selon l'invention comporte donc les opérations suivantes :
- la mise en place d'un premier étalon circulaire 32 entre la source de rayonnement 30 et le dispositif de détection 31 à N détecteurs,
- la réalisation d'un tour de scanner correspondant à m vues distinctes;
- la reconstruction de l'image de l'étalon 32 selon un procédé classique de manière à obtenir une image matricielle indiquant pour chaque point de coordonnées x et y la valeur de la brillance du pixel correspondant;
- le calcul, à l'aide de l'image matricielle, des N.m longueurs $d_{ij}$ des trajets des rayons X pour les N canaux et les m vues;
- le calcul (circuit 58) des atténuations théoriques et fictives $Ac_{ij}$ pour les trajets $d_{ij}$ et leur enregistrement dans une mémoire 60;
- le calcul (circuit 59) à l'aide de l'image matricielle des atténuations mesurées $Am_{ij}$ pour les mêmes trajets $d_{ij}$ en tenant compte des valeurs de brillance des pixels, et leur enregistrement dans une mémoire 61;
- le calcul (circuits 62 et 63) par canal, des valeurs moyennes $Ac_i$ et $Am_i$ des atténuations $Ac_{ij}$ et $Am_{ij}$;
- le calcul (circuits 64 et 67) d'un facteur de proportionnalité K entre les valeurs $Ac_i$ et $Am_i$; - le calcul des atténuations $K.Ac_i$ (circuit 65) et leur enregistrement dans une mémoire $66_1$ qui enregistre par ailleurs les atténuations correspondantes $Am_i$;
- la réitération des opérations ci-dessus après mise en place d'un deuxième étalon circulaire et l'enrégistrement des atténuations $K.Ac_i$ et $Am_i$ dans une mémoire $66_2$ et ainsi de suite pour d'autres étalons;
- l'étalonnage (circuit 68) à partir des atténuations $KAc_i$ et $Am_i$;

L'invention a été décrite en supposant que les étalons circulaires étaient centrés sur l'axe 33 de rotation du scanner; cependant, un tel centrage n'est pas nécessaire car les opérations effectuées à partir de l'image matricielle sont indépendantes des positions respectives des centres de l'étalon et du scanner.

L'invention a été décrite en se référant principalement au schéma de la figure 6 mais il est clair que les opérations et fonctions décrites peuvent être réalisées selon un système présentant un schéma fonctionnel différent sans sortir du cadre de la présente invention.

## Revendications

1. Procédé d'étalonnage d'un scanner à rayons X qui comporte une source de rayonnement (30) et un dispositif de détection (31) à N canaux, caractérisé en ce qu'il comprend les opérations suivantes :
- la mise en place d'un premier étalon circulaire (32) entre la source de rayonnement X (30) et le dispositif de détection (31),
- la réalisation d'un tour du scanner correspondant à m vues distinctes,
- la reconstruction de l'image (81) de l'étalon (32) de manière à obtenir une image matricielle indiquant pour chaque point de coordonnées x et y, la valeur de la brillance du pixel correspondant;
- le calcul, à l'aide de l'image matricielle des $N.m$ longueurs $d_{ij}$ des trajets des rayons X pour les N canaux et les m vues,
- le calcul des atténuations théoriques et fictives $Ac_{ij}$ pour les trajets $d_{ij}$ sachant que le coefficient d'absorption dans l'étalon a une valeur fixe et uniforme,
- le calcul, à l'aide de l'image matricielle, des atténuations mesurées $Am_{ij}$ pour les mêmes trajets $d_{ij}$ en tenant compte des valeurs de brillance des pixels,
- le calcul, par canal, des valeurs moyennes $Ac_i$ et $Am_i$ des atténuations $Ac_{ij}$ et $Am_{ij}$,
- le calcul d'un facteur de proportionnalité K entre les valeurs $Ac_i$ et $Am_i$,
- le calcul des atténuations $K.Ac_i$, et
- le calcul, à partir des valeurs $KAc_i$ et $Am_i$, des corrections à appliquer à chaque canal

2. Procédé d'étalonnage selon la revendication caractérisé en ce que les opérations sont répétées pour d'autres étalons de diamètres différents.

3. Procédé d'étalonnage selon la revendication 1 ou 2, caractérisé en ce que l'opération de calcul du facteur de proportionnalité K comporte une première opération de calcul de la moyenne des valeurs $Ac_i$ et $Am_i$ correspondant à un canal de rang i, une deuxième opération de comparaison entre les valeurs moyennes ainsi obtenues de manière à obtenir un coefficient de proportionnalité $K_i$ par canal et une troisième opération de calcul de la moyenne des coefficients $K_i$.

4. Système pour mettre en oeuvre le procédé d'étalonnage selon les revendications 1 à 3, dans un scanner comportant une source de rayonnement X (30) et un dispositif de détection (31), caractérisé en ce qu'il comprend :
- des moyens (56) pour réaliser une image matricielle de l'étalon (32) sous forme de coordonnées x et y de pixels et de valeurs de brillance desdits pixels,
- des premiers moyens de calcul (57) pour calculer, à partir de l'image matricielle de l'étalon, $N.m$ longueurs $d_{ij}$ de trajets des rayons X pour les N canaux et les m vues,
- des deuxièmes moyens de calcul (58 à 65, 67) pour calculer, d'une part, le coefficient de proportionnalité K entre les valeurs $Ac_{ij}$ et $AM_{ij}$, et, d'autre part, les valeurs $K.Ac_i$, et
- des moyens d'enregistrement (66) pour enregistrer les valeurs $K.Ac_i$ et les valeurs $Am_i$, et
- des troisièmes moyens de calcul (68) pour calculer, à partir des valeurs $KAc_i$ et $Am_i$, des corrections à effectuer par canal en fonction de l'atténuation.

FIG_1

FIG_2

Atténuation
(Echelle logarithmique)

N° du canal

Atténuation
(Echelle logarithmique)

FIG_3

FIG_4

FIG_7

FIG_5

FIG_6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | US-A-4 352 020 (I. HORIBA et al.) <br> * Résumé; colonne 3, lignes 20-52; colonne 5, lignes 13-38; colonne 6, ligne 56 - colonne 7, ligne 15; colonne 10, ligne 61 - colonne 11, ligne 25; colonne 11, ligne 37 - colonne 12, ligne 16; figures 1-3,13-17 * <br> --- | 1,4 | A 61 B 6/00 <br> A 61 B 6/03 |
| A | EP-A-0 218 367 (PICKER INTERNATIONAL INC.) <br> * Résumé; colonne 3, ligne 28 - colonne 4, ligne 15; colonne 5, ligne 39 - colonne 6, ligne 53; figures 1,2 * <br> --- | 1,4 | |
| A | EP-A-0 154 429 (K.K. TOSHIBA) <br> * Résumé; page 5, ligne 29 - page 6, ligne 7; page 6, ligne 24 - page 7, ligne 15; page 9, lignes 14-33; page 21, ligne 3 - page 23, ligne 25; figures 1-2B,9,10 * <br> --- | 1,4 | |
| A | FR-A-2 368 760 (PHILIPS) <br> * Page 3, lignes 24-37; page 4, lignes 20-34; page 5, ligne 16 - page 6, ligne 35; figures 1-4 * <br> --- | 1,3 | DOMAINES TECHNIQUES RECHERCHES (Int. Cl.5) <br><br> A 61 B |
| A | EP-A-0 089 096 (PHILIPS) <br> * Résumé; page 8, lignes 2-10; page 11, ligne 20 - page 13, ligne 1; page 15, ligne 30 - page 17, ligne 16; figures 1-3 * <br> ----- | 1-4 | |

Le présent rapport a été établi pour toutes les revendications

| Lieu de la recherche | Date d'achèvement de la recherche | Examinateur |
|---|---|---|
| LA HAYE | 22-12-1989 | RIEB K.D. |